(19) Europäisches Patentamt
European Patent Office
Office européen des brevets

(11) Publication number : 0 435 682 A2

# (12) EUROPEAN PATENT APPLICATION

(21) Application number : 90314360.0

(51) Int. Cl.$^5$ : A61K 31/557

(22) Date of filing : 28.12.90

(30) Priority : 28.12.89 US 458137

(43) Date of publication of application : 03.07.91 Bulletin 91/27

(84) Designated Contracting States : AT BE CH DE DK ES FR GB GR IT LI LU NL SE

(71) Applicant : ALLERGAN, INC.
2525 Dupont Drive, P.O. Box 19534
Irvine, California 92713-9534 (US)

(72) Inventor : Wheeler, Larry A.
18 Valley View
Irvine, California 92715 (US)

(74) Representative : Hutchins, Michael Richard et al
Graham Watt & Co. London Road Riverhead
Sevenoaks, Kent TN13 2BN (GB)

## (54) The use of inclusion complexes of prostaglandins with cyclodextrins in the treatment of ocular hypertension.

(57) This invention relates to the use of an inclusion complex formed from a cyclodextrin and a prostaglandin compound, which compound is capable of reducing intraocular pressure, for the manufacture of an ophthalmic medicament for the treatment of ocular hypertension.

EP 0 435 682 A2

Jouve, 18, rue Saint-Denis, 75001 PARIS

# THE USE OF INCLUSION COMPLEXES OF PROSTAGLANDINS WITH CYCLODEXTRINS IN THE TREATMENT OF OCULAR HYPERTENSION

## Field of the Invention

The present invention relates to the use of inclusion complexes of prostaglandins with cyclodextrins in the treatment of ocular hypertension. More particularly, this invention relates to a method of treating ocular hypertension by using inclusion complexes of prostaglandins capable of decreasing intraocular pressure with cyclodextrins. The present invention further relates to the use of such prostaglandin-cyclodextrin inclusion complexes in the preparation of pharmaceutical compositions useful in the treatment of ocular hypertension.

## Background of the Invention

"Prostaglandin" (with usual abbreviation "PG") is a general term used to designate a group of compounds which can be described as derivatives of prostanoic acid. The structural formula of prostanoic acid is as follows:

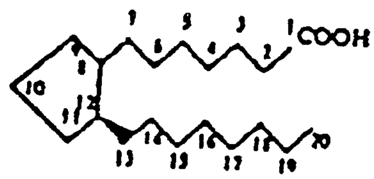

Various types of prostaglandins are known (including the types E, F, A, B, C, D), depending on the structure of and the substituents carried on the alicyclic ring of the prostanoic acid carbon skeleton. Further classification is based on the number of unsaturated bonds in the side chain indicated by numerical subscripts after the generic type of prostaglandin [e.g. prostaglandin $E_1$ ($PGE_1$), prostaglandin $E_2$ ($PGE_2$)], and on the configuration of the substituents on the alicyclic ring indicated by $\alpha$ or $\beta$ [e.g. prostaglandin $F_{2\alpha}$ ($PGF_{2\alpha}$)].

Prostaglandins were earlier regarded as potent ocular hypertensives, however, evidence accumulated in the last decade shows that some protaglandins are highly effective ocular hypotensive agents, and are ideally suited for the long-term medical management of glaucoma (see, for example, Bito, L. z. Biological Protection With Prostaglandins Cohen, M. M., ed., Boca Raton, Fla, CRC Press Inc., 1985, pp. 231-252 ; and Bito, L. Z., Applied Pharmacology in the Medical Treatment of Glaucomas Drance, S. M. and Neufeld, A. H. eds., New York, Grune & Stratton, 1984, pp. 477-505). Such prostaglandins include $PGF_{2\alpha}$, $PGF_{1\alpha}$, $PGE_2$, and certain lipid-soluble esters, such as 1-isopropyl ester of such compounds. The isopropyl ester of $PGF_{2\alpha}$ has been shown to have significantly greater hypotensive potency than the parent compound, presumably as a result of its more effective transfer through the cornea. This compound has recently been described as "the most potent ocular hypotensive agent ever reported" [(see, for example, Bito, L. Z., Arch. Ophthalmol. 105, 1036 (1987), and Siebold et al., Prodrug 5, 3 (1989)]. According to co-pending European Patent Application No. 90 30 8268.3 [corresponding to USSN Ser. No. 386,835 (filed July 27, 1989], the disclosure of which is hereby expressly incorporated by reference, certain 11-acyl-prostaglandins, such as 11-pivaloyl, 11-acetyl, 11-isobutyryl, 11-valeryl, and 11-isovaleryl $PGF_{2\alpha}$, are also potent hypotensives and are, therefore, useful in the management of glaucoma. Similarly, 11,15-9,16- and 9,11-diesters of prostaglandins, for example 11,15-dipivaloyl $PGF_{2\alpha}$ are known to have ocular hypotensive activity. See the co-pending European patent applications numbers 90 30 8269.1, 90 30 8289.9 and 90 30 8270.9 [corresponding to USSN Nos. 385,645, 386,312 and 385,834 (all filed July 27, 1989)], the disclosures of which are hereby expressly incorporated by reference. Although the precise mechanism is not yet known, recent experimental results indicate that the prostaglandin-induced reduction in intraocluar pressure results from increased oveoscleral outflow [Nilsson et al., Invest. ophthalmol. Vis. Sci. 28 (suppl), 284 (1987)].

Whereas prostaglandins appear to be devoid of significant intraocular side effects, ocular surface (conjunctival) hyperemia and foreign-body sensation have been consistently associated with the topical ocular use of such compounds, in particular $PGF_{2\alpha}$ and its prodrugs, e.g. its 1-isopropyl ester, in humans. The clinical potentials of prostaglandins in the management of conditions associated with increased ocular pressure, e.g. glaucoma are greatly limited by these side effects.

## Summary of the Invention

The present invention is based on the surprising discovery that if the prostaglandin compounds are applied to the eye as inclusion complexes with cyclodextrins (also referred to as "cyclodextrin clathrates"), their ability to decrease intraocular pressure is substantially increased, whereas the main side-effect, causing ocular surface hyperemia is greatly reduced. Although the underlying mechanism is not entirely understood yet, this favorable effect that greatly facilitates the clinical use of prostaglandins in the management of glaucoma, is probably due to the preferential delivery of the complexed prostaglandins to the cornea, versus the conjunctiva which contains the vascular receptors responsible for hyperemia.

In one aspect, the present invention relates to a method for treating ocular hypertension, by applying to the eye an ophthalmic formulation containing a therapeutically effective amount of at least one prostaglandin compound capable of reducing intraocular pressure, or a pharmaceutically acceptable salt thereof, in the form of its inclusion complex with one or more cyclodextrins.

In another aspect, the present invention relates to the use of inclusion complexes of prostaglandin compounds or pharmaceutically acceptable salts thereof with cyclodextrins in the preparation of pharmaceutical formulations for treating ocular hypertension, which comprises admixing a therapeutically effective amount of inclusion complex of at least one prostaglandin compound capable of reducing intraocular pressure, or a pharmaceutically acceptable salt thereof, with one or more cyclodextrins, with an ophthalmically acceptable excipient.

Cyclodextrins are cyclic molecules consisting of six, seven or eight D-glucopyranose units forming $\alpha$-1,4-glucoside units. $\alpha$, $\beta$ and gamma-cyclodextrins are composed of six, seven, and eight D-glucopyranose units, respectively. Cyclodextrins are of hydrophilic character on the outside, and are, therefore, water-soluble, and have a hydrophobic "pocket" (cavity) inside. Consequently, molecules which are less polar than water and are of appropriate shape and size to penetrate the cavity of the cyclodextrin molecule, generally complex with cyclodextrins, resulting inclusion complexes (cyclodextrin clathrates). Various cyclodextrin derivatives with improved properties, such as dimethylcyclodextrin, water-soluble polymers of cyclodextrins, products of the condensation of cyclodextrins with propylene oxide (2-hydroxypropylcyclodextrins), are also known in the art.

Cyclodextrins are widely used in the food industry, for example as food flavour stabilizers, and in the pharmaceutical industry, primarily for solubilization and stabilization of lipophilic drugs. Inclusion complexes of prostaglandins or their analogues with cyclodextrins are, for example, disclosed in the U. S. Patents Nos. 3,816,393; 4,054,736 ; 4,140,712 4,232,009 ; and 4,623,641. There is nothing in the art that would suggest that inclusion complexes of prostaglandins with cyclodextrins would have a substantially increased intraocular pressure-reducing activity. Similarly, nothing in the art suggests that prostaglandins and cyclodextrins interact in such a way that the accessability of prostaglandins to conjunctival vascular receptors that cause hyperemia, is masked, whereas their ability to decrease intraocular pressure is substantially enhanced. In view of the fact that both the cornea and conjunctiva are hydrophobic cell membranes, the preferential delivery of the cyclodextrin-prostaglandin inclusion complexes to the cornea is entirely unexpected.

## Detailed Description of the Invention

### 1. Definitions

The term "prostaglandin" and grammatical variants thereof, as used herein, refer to a group of naturally occurring or synthetic compounds which contain the carbon skeleton of prostanoic acid, their derivatives and analogues having a carbon skeleton similar to that of prostanoic acid, and derivatives of such analogues. The term includes "prodrugs" of prostaglandins, that are metabolized to provide pharmaceutically active prostaglandin compounds. The prostaglandins used according to the present invention have a functional definition, and are defined as prostaglandin compounds "capable of reducing intraocular pressure". The use of any such compound, known in the art or discovered hereinafter, in accordance with the appended claims, is within the scope of the present invention.

The term "cyclodextrin" as used herein, refers to $\alpha$ $\beta$ and gamma-cyclodextrins, and their derivatives, including but not limited to their substituted derivatives, water-soluble polymers of cyclodextrins, products of the condensation of cyclodextrins with propylene oxide (2-hydroxypropylcyclodextrins).

The term "inclusion complex" of prostaglandins with cyclodextrins is used to refer to complexes comprising cyclodextrin "host" molecules and prostaglandin "guests" included in the cavity of the cyclodextrin molecules. The mutual affinity between the guest and host is reflected by the dissociation constants of such complexes, which is the concentration at which the complex is about half dissociated. The dissociation constant is a function of the specific cyclodextrin type and of the included cyclodextrin molecule.

The term "therapeutically effective amount" is used to indicate an amount of cyclodextrin that is capable of reducing intraocular pressure when applied to the eye in the form of its cyclodextrin complex.

2. Description of Preferred Embodiments

For the purpose of the present invention, cyclodextrin compounds capable of reducing intraocular pressure are complexed with cyclodextrin compounds, and the obtained inclusion complexes are applied to the eye in therapeutically effective amounts. It has been experimentally found that the prostaglandin compounds, when applied to the eye in the form of inclusion complexes with cyclodextrins, substantially increase their intraocular pressure-reducing activity, and at the same time, cause significantly lower and shorter-lasting ocular surface hyperemia than when applied in uncomplexed form.

Preferably, prostaglandin compounds that are known to have strong ocular hypotensive activity are used in accordance with the present invention. Particularly preferred are prostaglandins compounds of the D, E and F types, such as $PGE_2$, $PGF_{2\alpha}$ and their derivatives, preferably 11-monoesters, and 11,15- 9,15-, and 9,11-diesters, such as 11,15-dipivaloyl esters of $PGF_{2\alpha}$ Also preferred are lipid-soluble carbon 1-esters of prostaglandins, in particular, $PGF_{2\alpha}$-1-isopropyl ester. Such prostaglandin compounds are known in the art, and are commercially available or can be prepared from commercially available compounds in a conventional manner.

For the inclusion of prostaglandin compounds, α-, β- and gamma-cyclodextrins and mixtures thereof may be equally used. Preferred are derivatives of the parent cyclodextrins, exhibiting improved solubility, including 2-hydroxypropylcyclodextrins. These compounds are, for example, described in the U. S. Patent No 3,459,731. Presently, only 2-hydroxypropyl-β-cyclodextrin is commercially available, however, the corresponding α- and gamma-homolog are also well known [see e.g. Pitha, J., Neurotransmissions V(1), 1-4 (1989)].

The inclusion complexes of prostaglandins with cyclodextrins may be prepared by methods known in the art. Usually, in preparing the inclusion complexes, cyclodextrin is dissolved in water, in an aqueous buffer solution, and/or in a water-miscible organic solvent, simply by stirring briefly at ambient or slightly elevated temperature. For example, 2-hydroxypropylcyclodextrins of suitable average degree of substitution (about 4 to 8 hydroxypropyl groups per cyclodextrin molecule) are readily soluble in aqueous media, in 95% ethanol or dimethyl sulfoxide.

The obtained clear solution may then be affixed with a solution of the desired prostaglandin compound in a water-miscible organic solvent. The ratios of the two solutions may vary according to the solubilities of the starting materials and of the products. The inclusion complexes are usually formed upon heating the mixture till the formation of a clear solution. Preferably, the temperature is not allowed to exceed about 70°C, during the preparation of cyclodextrin inclusion complexes.

Alternatively, the prostaglandin compound to be complexed may be pulverized and added, in excess, to the clear solution of the cyclodextrin compound, and the obtained suspension is stirred at ambient temperature. The dissolution of a drug, e.g. a prostaglandin compound, is usually linearly proportional to the concentration of the employed cyclodextrin solution. Generally, about 0.05 to 50%, preferably about 1% to 50%, more preferably about 5% to 50% cyclodextrin concentrations are recommended. Hydrophobic interactions drive the prostaglandin molecule into the cavity of the cyclodextrin, forming the guest-host inclusion complex. The equilibrium dissolution may take from several minutes to several hours to establish. Thereafter, the remaining, uncomplexed prostaglandin compound may be removed, for example by centrifugation or ultrafiltration, which clarifies and sterilizes the solution.

The obtained solutions of the prostaglandin-cyclodextrin complexes may be directly suitable for medical purposes, or the complexed drug can be precipitated from the solutions by conventional techniques, for example by cooling, or by concentration under reduced pressure. The complexed prostaglandins can also be freeze dried, and the obtained powder may be manufactured into solid pharmaceutical preparations, e.g. tablets. The solid pharmaceutical preparations usually have much higher stability than the uncomplexed drugs, and fully dissolve in water within minutes.

Cyclodextrins have been excessively tested for safety in various animal models and clinical trials, and have been found to be without any significant side-effects. 20% (w/w) solutions of 2-hydroxypropyl-β-cyclodextrin caused no irritation on rabbit eyes [Pitha, J., Supra].

Pharmaceutical compositions may be prepared by combining a therapeutically efficient amount of an inclusion complex of at least one prostaglandin compound, or a pharmaceutically acceptable salt thereof, with at least one cyclodextrin, as an active ingredient, with conventional pharmaceutical excipient. For ophthalmic application, preferably solutions are prepared using a physiological saline solution as a major vehicle. The pH of such ophthalmic solutions should preferably be maintained between 6.5 and 7.2 with an appropriate buffer system. The formulations may also contain conventional, pharmaceutically acceptable preservatives and stabilizers.

Preferred preservatives that may be used in the pharmaceutical compositions used according to the present invention include, but are not limited to, benzalkonium chloride, chlorobutanol, thimerosal, phenylmercuric acetate and phenylmercuric nitrate.

Likewise, various preferred vehicles may be used in the ophthalmic preparations used in the methods of the present invention. These vehicles include, but are not limited to, polyvinyl alcohol, povidone, hydroxypropyl methyl cellulose, poloxamers, carboxymethyl cellulose, hydroxyethyl cellulose and purified water.

Tonicity adjustors may be added as needed or convenient. They include, but are not limited to, salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. Accordingly, buffers include acetate buffers, citrate buffers, phosphate buffers and borate buffers. Acids or bases may be used to adjust the pH of these formulations as needed.

In a similar vein, an opthalmically acceptable antioxidant for use in the present invention includes, but is not limited to, sodium metabisulfite, sodium thiosulfate, acetylcysteine, butylated hydroxyanisole and butylated hydroxytoluene.

Other excipient components which may be included in the ophthalmic preparations are chelating agents. The preferred chelating agent is edentate disodium, although other chelating agents may also be used in place or in conjunction with it.

The ingredients are usually used in the following amounts :

| Ingredient | Amount (% w/v) |
|---|---|
| Prostaglandin (as inclusion complex) | 0.1-5 |
| Preservative | 0-0.10 |
| Vehicle | 0-40 |
| Tonicity Adjustor | 1-10 |
| Buffer | 0.01-10 |
| pH Adjustor | q.s. pH 4.5-7.5 |
| Antioxidant | as needed |
| Purified Water | as needed to make 100% |

The therapeutically effective concentrations of the complexed prostaglandin compounds in their topically applicable solutions usually are in the range of about 0.001% to about 5%, preferably between about 0.01% to about 5%, more preferably between about 0.1% to about 1%. The actual dose depends on the specific prostaglandin compound and cyclodextrin, and on the condition to be treated ; and the selection of the appropriate dose is well within the knowledge of the skilled physician.

The invention is further illustrated by the following non-limiting Example.

3. Example

Prostaglandin $F_{2\alpha}$ TRIS salt was dissolved in either distilled water or in a 5% aqueous hydroxypropyl-β-cyclodextrin solution, to provide 0.1% (w/v) solutions. Two groups of experimental New Zealand Albino rabbits, containing 6 and 8 rabbits, respectively, were treated by administering 25μℓ of each solution to the surface of one eye. The contralateral eye received vehicle as a control. Intraocular pressure (IOP) was measured by applanation pneumatonometry model (Model 3ORT, manufactured by Digilab) at the time of administration, and at intervals of 1, 2, 3, 4, and 6 hours thereafter. The data obtained are set forth in Table I.

The data clearly show that complexation with β-cyclodextrin has substantially increased the intraocular pressure-lowering effect of $PGF_{2\alpha}$. At the same time, the percentage of experimental animals that exhibited ocular surface hyperemia three hours and later following administration, was significantly lower when the prostaglandin compound was administered as an inclusion complex with β-cyclodextrin.

The foregoing description details specific methods that can be employed to practice the present invention and represents the best mode contemplated. However detailed the foregoing may appear in text, it should not be construed as limiting the overall scope hereof ; rather, the ambit of the present invention is to be governed only by the lawful construction of the appended claims.

In addition to the foregoing embodiments, it will be understood that the invention also comprehends a method for manufacturing an ophthalmic medicament, which method comprises bringing an inclusion complex

of a cyclodextrin and an appropriate prostaglandin into association with an ophthalmically acceptable carrier and, where necessary, adjusting the pH to a value within the range 4.5-7.5 and/or adding an ophthalmically acceptable preservative and optionally other ingredients as defined herein ; and filling the resultant composition into an appropriate container, for example a dispensing container (e.g. for dispensing eye drops or an ointment).

TABLE I

INTRAOCULAR PRESSURE (mmHg) CHANGES AT PREDETERMINED TIMES (HR) AFTER PROSTAGLANDIN ADMINISTRATION

| PROSTAGLANDIN | | CHANGES IN INTRAOCULAR PRESSURE (mmHg) AT PREDETERMINED TIMES (HR) | | | | | |
|---|---|---|---|---|---|---|---|
| | | 0.5 | 1.0 | 2.0 | 3.0 | 4.0 | 6.0 |
| Prostaglandin $F_{2\alpha}$ (in 5% hydroxypropyl-β-cyclodextrin) | 0.1% | - | -0.6 | -11.2** | -8.1** | -8.0** | -5.7** |
| Prostaglandin $F_{2\alpha}$ (in aqueous) | 0.1% | - | -6.3* | -3.0 | -3.0 | -1.5 | 0 |

| PROSTAGLANDIN | | PERCENT ANIMALS EXHIBITING OCULAR SURFACE HYPEREMIA % HYPEREMIA AT PREDETERMINED TIMES (HR) | | | | | |
|---|---|---|---|---|---|---|---|
| | | 0.5 | 1.0 | 2.0 | 3.0 | 4.0 | 6.0 |
| Prostaglandin $F_{2\alpha}$ in 5% hydroxypropyl-β-cyclodextrin | 0.1% | - | 100 | 100 | 66 | 66 | 33 |
| Prostaglandin $F_{2\alpha}$ (in aqueous) | 0.1% | - | 100 | 100 | 87 | 100 | 75 |

* $p < 0.05$, ** $p < 0.01$ according to Students paired t test

## Claims

1. The use of an inclusion complex formed from a cyclodextrin and a prostaglandin compound, which compound is capable of reducing intraocular pressure, for the manufacture of an ophthalmic medicament for the treatment of ocular hypertension.

2. The use according to claim 1, wherein said prostaglandin compound is selected from the group consisting of prostaglandins of the D, E, and F types (such as $PGE_2$, $PGE_{2\alpha}$, and their derivatives) and pharmaceutically acceptable salts thereof.

3. The use according to claim 2, wherein said derivative is selected from the group consisting of 11-monoesters, 11,15- 9,15-, and 9,11-diesters, and lipid-soluble carbon 1-esters of $PGF_{2\alpha}$, said derivative for example being $PGF_{2\alpha}$ 11,15-dipivaloyl ester.

4. The use according to any one of the preceding claims, wherein said inclusion complex is formed with a cyclodextrin selected from the group consisting of α-, β- and gamma-cyclodextrin.

5. The use according to any one of claims 1 to 3, wherein said cyclodextrin is a 2-hydroxypropyl cyclodextrin, such as 2-hydroxypropyl-β-cyclodextrin.

6. The use according to any one of the preceding claims, wherein said ophthalmic formulation is an aqueous solution containing from about 0.001% to about 5% (w/w) (for example about 0.1% to about 1%) of said cyclodextrin compound or pharmaceutically acceptable salt thereof.

7. A pharmaceutical composition adapted for topical ophthalmic administration to the exclusion of administration orally or by injection, said composition comprising an inclusion complex of a cyclodextrin and a prostaglandin compound as defined in any one of the preceding claims and an ophthalmically acceptable carrier.

8. An ophthalmic medicament comprising a dispensing container (e.g. for dispensing eyedrops or an ointment) adapted for dispensing an ophthalmic composition to the eye and, contained therein, an ophthalmic composition containing an inclusion complex of a cyclodextrin and a prostaglandin compound as defined in any one of claims 1 to 6, and an ophthalmically acceptable carrier.

9. An aqueous ophthalmic solution, suitable for use in the treatment of ocular hypertension, which solution has a pH in the range 4.5-7.5 and comprises from 0.1 to 5% (w/v) of an inclusion complex of a cyclodextrin and a prostaglandin compound as defined in any one of claims 1 to 6 ; an ophthalmically acceptable preservative in an amount of up to 0.1% (w/v), 0-40% (w/v) of an ophthalmically acceptable vehicle ; 1-10% (w/v) of a toxicity adjuster ; 0.01-10% (w/v) of a buffering agent ; and sufficient purified water to make 100% (w/v).

10. A composition, medicament or solution as defined in any one of claims 7 to 9 wherein the prostaglandin is prostaglandin $F_{2\alpha}$ and the cyclodextrin is hydroxypropyl-β-cyclodextrin.